# FASCICULE DE BREVET EUROPEEN

(11) **EP 1 551 992 B1**
(45) Date de publication et mention de la délivrance du brevet: **27.12.2006**
(21) Numéro de dépôt: 03778411.3
(22) Date de dépôt: 20.10.2003
(51) Int. Cl.: C12Q 1/02

(54) **CRIBLAGE DE MOLECULES A ACTIVITE ANTI-PRION : KITS, METHODES ET MOLECULES CRIBLEES**
REIHENTESTVERFAHREN AUF MOLEKÜLE MIT ANTI-PRIONAKTIVITÄT: KITS, METHODEN UND MOLEKÜLE
SCREENING MOLECULES WITH ANTI-PRION ACTIVITY: KITS, METHODS AND SCREENED MOLECULES

(30) Priorité: 18.10.2002 FR 0213022; 07.07.2003 FR 0308289
(43) Date de publication de la demande: 13.07.2005
(73) Titulaire: CENTRE NATIONAL DE LA RECHERCHE SCIENTIFIQUE (CNRS), 75794 Paris Cedex 16 (FR); Université Victor Segalen, 33076 Bordeaux (FR); UNIVERSITE DE POITIERS, 86034 Poitiers Cedex (FR)
(72) Inventeur: BLONDEL, Marc, F-29250 Saint Pol de Leon (FR); BACH, Stéphane, F-29250 Saint Pol de Leon (FR); CULLIN, Christophe, F-33700 Merignac (FR); TALAREK, Nicolas, Res La Malvoisie, F-33400 Talence (FR); VIERFOND, Jean-Michel, F-94700 Maisons Alfort (FR); METTEY, Yvette, F-86000 Poitiers (FR)
(74) Mandataire: Peaucelle, Chantal
(86) Numéro de dépôt international: PCT/FR2003/003101
(87) Numéro de publication internationale: WO 2004/035813

(56) Documents cités:
- WO-A-99/29891
- WO-A-02/065136
- US-A- 3 758 479
- US-A- 4 024 242
- US-A- 5 695 782
- WEISSMAN J ET AL: "Mechanism of amyloid formation and propagation: Lessons from a yeast prion." BIOPHYSICAL JOURNAL, vol. 80, no. 1 Part 2, janvier 2001 (2001-01), page 329a, XP002237383 45th Annual Meeting of the Biophysical Society;Boston, Massachusetts, USA; February 17-21, 2001 ISSN: 0006-3495
- WICKNER R B ET AL: "Prions of yeast, [PSI] and [URE3], as models for neurodegenerative diseases" COLD SPRING HARBOR SYMPOSIA ON QUANTITATIVE BIOLOGY 1996 UNITED STATES, vol. 61, 1996, pages 541-550, XP001098910 ISSN: 0091-7451
- DATABASE BIOSIS [Online] BIOSCIENCES INFORMATION SERVICE, PHILADELPHIA, PA, US; juin 2000 (2000-06), TALLOCZY ZSOLT ET AL: "The (KIL-d) element specifically regulates viral gene expression in yeast." XP002237388 Database accession no. PREV200000387721 & GENETICS, vol. 155, no. 2, juin 2000 (2000-06), pages 601-609, ISSN: 0016-6731
- METTEY Y ET AL.: "Synthesis of 11-Aminodibenzo[b,f]thiazepines and Fluoro Derivatives" HETEROCYCLIC CHEM, vol. 34, 1997, pages 465-467, XP009008912
- S.V. KESSAR ET AL: "New Routes to Condensed Polynuclear Compounds, II Direct Benzyne Cyclisation of N-Chlorobenzylidene Arylamines" TETRAHEDRON LETTERS, 1969, pages 1155-1156, XP009009071 cité dans la demande
- KORTH CARSTEN ET AL: "Acridine and phenothiazine derivatives as pharmacotherapeutics for prion disease." PROCEEDINGS OF THE NATIONAL ACADEMY OF SCIENCES OF THE UNITED STATES, vol. 98, no. 17, 14 août 2001 (2001-08-14), pages 9836-9841, XP002237386 August 14, 2001 ISSN: 0027-8424 cité dans la demande
- EMTER ROGER ET AL: "ERG6 and PDR5 regulate small lipophilic drug accumulation in yeast cells via distinct mechanisms." FEBS LETTERS, vol. 521, no. 1-3, 2002, pages 57-61, XP002237387 19 June, 2002 ISSN: 0014-5793
- DATABASE EMBASE [Online] ELSEVIER SCIENCE PUBLISHERS, AMSTERDAM, NL; 1984, CROWLEY J C ET AL: "Molecular cloning of chromosome I DNA from Saccharomyces cerevisiae: Isolation of the ADE1 gene" XP002273243 Database accession no. EMB-1984159946 & JOURNAL OF BACTERIOLOGY 1984 UNITED STATES, vol. 159, no. 1, 1984, pages 413-417,

## Description

La présente invention se rapporte à du criblage de molécules à activité anti-prion. Elle vise plus particulièrement des kits de criblage de molécules à activité anti-prion, les méthodes de criblage, et une famille de molécules à activité anti-prion mise en évidence à l'aide du crible selon l'invention.

Les prions sont des protéines infectieuses responsables chez les mammifères de certaines maladies neuro-dégénératives de type encéphalopathies spongiformes comme la maladie de Creutzfeldt-Jakob chez l'homme ou encore les maladies dites « de la vache folle » chez les bovins ou « tremblante du mouton » chez les ovins. Ces différentes maladies sont provoquées par des agents infectieux non conventionnels : à la différence des agents infectieux traditionnels (bactéries, virus par exemple), ils ne contiennent pas d'acides nucléiques. Le Professeur Stanley Prusiner a formulé l'hypothèse de « la protéine seule », selon laquelle l'agent infectieux ne serait constitué que d'une protéine. Cette protéine existe naturellement dans les cellules sous une forme « normale » (ou PrP^{c}), c'est-à-dire soluble, essentiellement sous forme d'hélice α et non agrégée donc fonctionnelle. Dans certaines conditions encore inconnues, cette protéine peut se transformer en une forme prion (ou PrP^{SC}). Sous cette forme prion, la protéine forme des agrégats insolubles, essentiellement sous forme de feuillets β. Le caractère infectieux de cette conformation prion PrP^{sc} viendrait du fait que, outre les caractéristiques indiquées précédemment, la protéine sous forme prion gagne également la capacité à catalyser le passage de la forme cellulaire normale PrP^{c} vers la forme prion PrP^{sc} dans un mécanisme de type « boule de neige ».

La levure de boulanger *Saccharomyces cerevisiae* contient plusieurs protéines se comportant comme des prions (Fernandez-Bellot et Cullin, 2001). Dès les années soixante, deux mécanismes génétiques non conventionnels y sont décrits. En 1994, les phénotypes correspondants [PSI+] et [URE3] ont été proposés comme résultant de l'inactivation auto-catalytique des protéines Sup35p et Ure2p respectivement. Ces protéines prions présentent donc a priori une analogie mécanistique avec les systèmes mammifères délétères pour la santé publique. A l'instar de la protéine PrP, la protéine Sup35p « normale » passe d'un état soluble à un état insoluble et agrégé dès que la protéine est en contact avec une autre protéine Sup35p sous la forme prion. Cet état agrégé est vérifié tant par des expériences de centrifugation que par des expériences de localisation intracellulaire. Les prions de la levure peuvent être éliminés (« curés ») par une forte dose (1 à 5 mM) de chlorure de guanidium. Suite à un tel traitement (qui doit être appliqué sur au moins six à dix générations), les agrégats protéiques générés par la présence des prions disparaissent et la protéine en question (Sup35p, par exemple) se retrouve sous une forme normale, soluble, fonctionnelle mais ayant conservé la susceptibilité d'être convertie sous une forme prion si elle se retrouvait à nouveau en contact avec une autre protéine Sup35p dans un tel état.

La protéine Sup35p, en complexe hétérodimérique avec la protéine Sup45p, forme un facteur de terminaison de la traduction. Ce facteur reconnaît les codons stop opales (UGA). Sous sa forme cellulaire normale (soluble et active) dans les souches [*psi*-], Sup35p, en association avec Sup45p termine efficacement la traduction au niveau de ces codons opales. Dans une souche [*PSI+*] où la protéine Sup35p est sous forme prion, elle est majoritairement présente sous forme d'agrégats insolubles. Ne pouvant pas se lier à Sup45p, elle est ainsi non fonctionnelle dans la terminaison de la traduction. Une petite fraction de l'ensemble des protéines Sup35p cellulaires reste toutefois soluble dans ces cellules [*PSI*+] où elle permet, en complexe avec Sup45p, d'assurer un « service minimum » de terminaison de la traduction, service essentiel à la survie de la levure. Un système colorimétrique permettant de détecter, de façon indirecte, la forme sous laquelle la protéine Sup35p est présente : normale ou prion, a été élaboré à partir de ces constatations. Ce système, décrit depuis longtemps (voir l'article de synthèse par Fernandez-Bellot et Cullin, 2001), est basé sur l'utilisation de l'allèle *ade1-14* du gène *ADE1,* codant pour une enzyme de la voie de biosynthèse de l'adénine : la SAICAR synthétase. Cette enzyme catalyse la formation de 4-(N-succinocarboxamide)-5-aminoimidazole ribonucléotide (SAICAR) à partir de 4-carboxy-5-aminoimidazole ribonucléotide (CAIR). L'allèle *ade1-14* contient un codon opale dans le cadre de lecture du gène *ADE1*. Dans une souche [psi-], Sup35p en association avec Sup45p va donc arrêter la traduction du gène *ADE1* au niveau de ce codon stop. La protéine ade1-14p ainsi traduite sera tronquée et donc non fonctionnelle. En conséquence les substrats en amont de l'enzyme Ade1p vont s'accumuler, notamment la 5-aminoimidazole ribonucléotide (AIR). L'AIR étant oxydé en un composé de couleur rouge, les colonies formées par les cellules [psi-] seront de couleur rouge. En outre, ces cellules seront auxotrophes pour l'adénine. A l'inverse, dans une souche [*PSI+*], la protéine Sup35p est essentiellement présente sous forme d'agrégats donc incapable de s'associer avec Sup45p pour stopper la traduction au niveau du codon opale de l'allèle *ade1-14* du gène *ADE1*. En conséquence, les ribosomes vont faire une pause au niveau de ce codon stop avant de reprendre leur activité de traduction (translecture). Une certaine quantité de protéine Ade1p fonctionnelle sera donc synthétisée, les cellules seront autotrophes pour l'adénine et formeront des colonies de couleur blanche à rosée.

Dans un article paru dans P.N.A.S, l'équipe du Pr. Stanley Prusiner divulgue un test de détection de molécules à activité anti-prion (Korth et al., 2001). Ce test est effectué sur un modèle de mammifères (neuroblastomes murins infectés par PrP^{sc}). Les conditions de sécurité (laboratoire P3) et de cultures cellulaires (manipulations assez lourdes) ne permettent pas de réaliser du criblage à haut débit.

La demande WO 98/30909 décrit également un procédé de criblage de molécules à activité anti-prion réalisé sur des rongeurs infectés par un agent transmissible non conventionnel. Cette méthode de criblage présente les mêmes limites que la méthode décrite dans P.N.A.S.

Les documents WO 02/065136 et WO 99/29891 décrivent des méthodes de criblage de molécules à activité anti-prion mettant en oeuvre une levure.

Les travaux des inventeurs les ont amenés à réaliser un système de criblage haut débit pour mettre en évidence des molécules possédant une activité anti-prion, basé sur le système rapporteur colorimétrique de la protéine Sup35p, décrit ci-dessus.

La présente invention est donc relative à un kit criblage de molécules à activité anti-prion, caractérisé en ce qu'il comporte en combinaison une levure de phénotype [*PSI*+], un antibiogramme et un agent de curage des prions à des doses sub-efficaces, ladite levure présentant l'allèle *ade1-14* du gène *ADE1* ainsi qu'un gène *ERG6* inactivé.

Bien que basé sur les prions des levures, le kit selon l'invention permet d'isoler des molécules actives contre les prions de mammifères. L'exemple 7 suivant montre que les molécules les plus actives isolées par le Pr. Prusiner

présentent également une activité dans le crible selon l'invention.

Pourtant, on observe de nombreuses différences entre les prions de levure et les prions de mammifères. Dans un article de la revue « Cellular and Molecular Life Sciences », le Professeur C.Cullin propose même au vu de ces différences de distinguer les prions de levures de ceux des mammifères en employant le terme de « propagons ». Comme différences notables entre les « prions » (mammifères) et les « propagons » (levure), on peut citer le caractère cytoplasmique des propagons alors que le prion PrP des mammifères est une protéine ancrée à la membrane plasmique, le caractère pathologique des prions de mammifères, ainsi qu'un certain nombre de différences biophysiques (structure ternaire et quaternaire, réversibilité du curage...)

L'un des principaux avantages d'un tel crible réside dans sa parfaite innocuité ce qui permet de le réaliser dans un laboratoire de biologie moléculaire classique de niveau L2, et non, comme requis dans les techniques antérieures, dans un laboratoire de niveau P3.

De plus, la grande facilité d'utilisation et le très faible coût de ce kit rendent le criblage à haut débit réalisable. L'utilisation de pastilles à antibiogramme, qui permettent la diffusion du produit en créant un gradient de concentration, permet en outre de tester en une seule expérience une multiplicité de concentrations, contrairement aux tests classiques, dans lesquels seule une concentration est testée. Pour chaque molécule dont on teste l'activité anti-prion, l'utilisation de l'antibiogramme permet également d'avoir des informations sur la toxicité du produit ainsi que sur le rapport activité/concentration, et de déterminer ainsi la meilleure concentration efficace.

La souche [*PSI*+] utilisée dans le kit selon l'invention porte une inactivation du gène *ERG6.* En effet, les levures sont naturellement assez peu perméables. En particulier, la levure préférée pour la mise en oeuvre de l'invention, *Saccharomyces cerevisiae,* présente une imperméabilité telle que la réalisation d'un criblage s'avère particulièrement peu efficace sans cette inactivation.

La méthode d'analyse du crible selon l'invention est visuelle grâce à l'utilisation de l'allèle ade1-14. Selon l'activité anti-prion de la molécule testée, les colonies de cellules auront une coloration rouge, rosée ou blanche. Le choix de la souche de levure peut permettre d'améliorer le contraste entre les colonies. En effet, certaines souches dites « Strong » facilitent l'analyse visuelle du crible. De telles souches possèdent un fort niveau d'agrégation des formes prions. A contrario, la souche sera dite « Weak ». Les souches préférées pour la mise en oeuvre de l'invention sont donc les souches de type « Strong ».

D'autres levures peuvent également être utilisées. On citera à titre d'exemples : *Kluyveromyces lactis, Pichia methanolica*, *Saccharomyces ludwigii, Kluyveromyces marxianus, Pichia pastoris, Zygosaccharomyces rouxi, Schizosaccharomyces pombe.*

Etant donné la létalité synthétique observée entre l'inactivation du gène *ERG6* et l'inactivation du gène *TRP1,* le gène *ERG6* pourra être délété en utilisant le gène *TRP1* comme marqueur de délétion.

Avantageusement, le kit comporte en outre un agent de curage des prions à doses sub-efficaces.

Par curage, on entend une élimination des formes prions dans les cellules de levure. Cette élimination peut être temporaire ou définitive.

A titre d'exemple, un agent de curage pour le prion peut être l'eau oxygénée ou préférentiellement, le chlorure de guanidium.

Par doses sub-efficaces, on entend des doses qui utilisées seules ne suffiraient pas à curer les prions des levures. Les valeurs de telles doses sont données, dans les exemples qui suivent, pour le chlorure de guanidium.

Les intérêts de la présence d'un agent de curage à des doses sub-efficaces sont de renforcer la sensibilité du crible et d'obtenir un meilleur contraste.

Le kit selon l'invention peut être mis en oeuvre dans une méthode de criblage de molécules à activité anti-prion. Cette méthode de criblage, également visée par l'invention, est caractérisée en ce qu'elle met en oeuvre une levure de phénotype [*PSI+*] présentant l'allèle *ade1*-*14* du gène ADE1 ainsi qu'un gène *ERG6* inactivé et comporte les étapes suivantes :
a. réalisation d'un tapis de cellules in vitro sur un milieu complémenté d'une dose sub-efficace d'un agent de curage des prions,
b. dépôt des composés à tester selon la méthode de l'antibiogramme,
c. incubation pendant environ 2-4 jours à environ 20-25°C, et,
d. analyse de la coloration des colonies cellulaires.

Cette méthode possède des avantages analogues à ceux du kit selon l'invention. Il s'agit d'un test visuel, très facile à analyser. Sa mise en oeuvre est très simple et peu onéreuse. Les précautions relatives à la sécurité sont celles d'un laboratoire classique de biologie moléculaire. Elle permet le criblage massif : une personne seule peut cribler manuellement plus de 400 produits par jour. Un criblage de très haut débit serait possible par automatisation de la méthode. Le résultat du crible est révélé au bout de 7 jours, sans qu'il soit nécessaire de recourir à des manipulations lourdes entre le jour J et le jour J+7 (éventuellement un changement de température de l'incubateur). Enfin, cette méthode est particulièrement économique.

Une des levures préférées pour la mise en oeuvre de cette méthode est *Saccharomyces cerevisiae.*

Avantageusement, l'agent de curage de l'étape a. est le chlorure de guanidium.

La méthode peut également comporter les étapes suivantes :
e. incubation pendant environ 2-4 jours à environ 2-6°C, et/ou,
f. réalisation d'un test de criblage secondaire.

L'incubation à 2-6°C permet d'accentuer le contraste des colorations des colonies.

Préférentiellement, le test de criblage secondaire pourra comporter les étapes suivantes :
- construction d'une souche de levure dans laquelle le gène *ADE2* est sous le contrôle du promoteur du gène *DAL5*
- réalisation des étapes a. à e. des méthodes décrites ci-avant.

Un tel crible secondaire permet de tester très rapidement si les molécules isolées lors du crible primaire peuvent avoir un effet général sur les prions chez la levure. En effet, les gènes *SUP35* (responsable du prion [*PSI+*]) et *URE2* (responsable du prion *[URE3])* codent pour des enzymes ayant des fonctions totalement différentes et dont les séquences primaires sont très éloignées.

L'invention couvre également les molécules isolées par la méthode de criblage selon l'invention.

En particulier, la méthode de criblage a permis d'isoler des agents anti-prion ayant la formule (I) suivante : 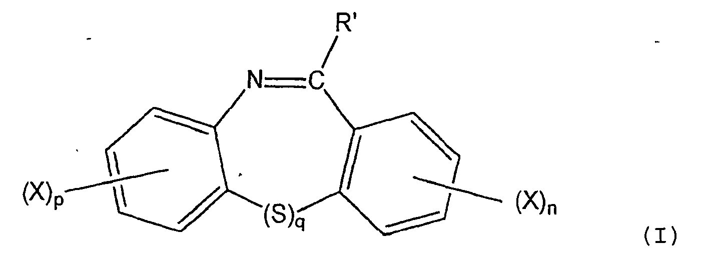 dans laquelle R' est un groupement H, NH₂, NHR², où R² est une chaîne alkyle ou alkylaminoalkyle de 1 à 10 carbones, ramifiée ou non,
X représente F, Cl, Br, I, CF₃, SR³, OR³, OH, NO₂, COR³, CONH₂, COOH, COOR³, où R³ est un groupement alkyl de 1 à 4 carbones, de préférence CH₃.
p et n, identiques ou différents, égalent 0, 1 ou 2,
q égale 0 ou 1.

L'invention couvre en particulier les agents anti-prion de formule (III): 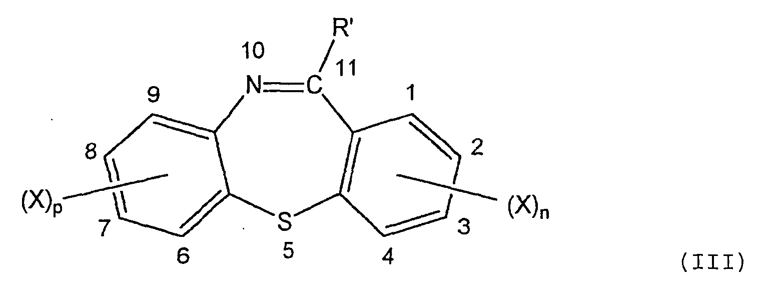 dans laquelle R' représente un groupement H, NH₂, NH- (CH₂)₃-N(CH₃)₂, NH-CH(CH₃)-(CH₂)₃-N(CH₂-CH₃)₂,
X représente F, Cl, CF₃,
p et n, identiques ou différents, égalent 0, 1 ou 2.

Cette famille de molécules, appelée « Kastellpaolitines » par les inventeurs, possède à un degré plus ou moins fort l'activité anti-prion recherchée. En particulier, les dérivés chlorés de cette famille sont particulièrement efficaces. Les meilleures efficacités sont obtenues lorsque le chlore est placé en position 2, 3, 4, de préférence, en position 4 (voir KP1 dans les exemples qui suivent).

L'invention vise plus particulièrement les composés de formule (II) : 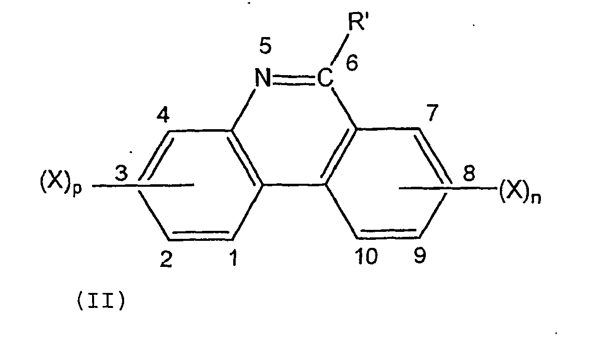 dans laquelle R' représente un groupement H, NH₂, NH-(CH₂)₃-N (CH₃) ₂, NH-CH (CH₃) - (CH₂) ₃-N (CH₂-CH₃)₂,
X représente F, Cl, CF₃,
p et n, identiques ou différents, égalent 0, 1 ou 2,
pour une utilisation comme médicament, et en particulier, comme agent anti-prion.

Elle vise également les compositions pharmaceutiques comprenant une quantité thérapeutiquement efficace d'au au moins un composé de formule (II) dans laquelle : 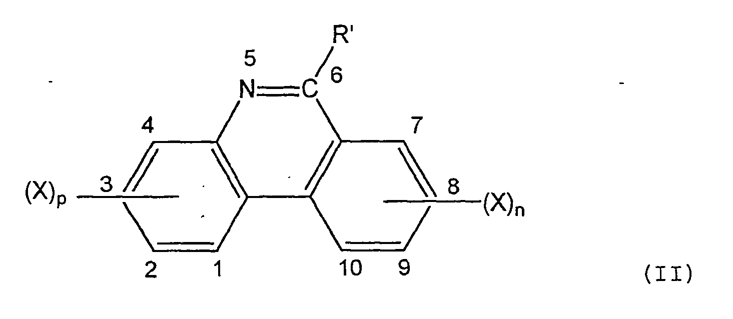 R' représente un groupement H, NH₂, NH-(CH₂)₃-N(CH₃)₂, NH-CH (CH₃) - (CH₂)₃ - N (CH₂-CH₃)₂,
X représente F, Cl, CF₃,
p et n, identiques ou différents, égalent 0, 1 ou 2, en association avec au moins un véhicule pharmaceutiquement acceptable.

Certains composés de cette famille sont particulièrement actifs. Il s'agit de la phénanthridine et de la 6-aminophénanthridine, ainsi que de leurs dérivés chlorés, en particulier lorsque le chlore est placé en position 8, 9, 10, de préférence, en position 10 (voir dans les exemples qui suivent).

Préférentiellement, dans les formules (II) et (III), R' représente NH₂. En effet, une très bonne activité des molécules a été constatée lorsque R' représente NH₂.

L'invention propose également une méthode de traitement des maladies neurodégénératives impliquant des agrégats protéiques, comportant une étape d'administration à un animal ou à un patient d'une quantité thérapeutiquement efficace d'au moins un des composés de formule (I), (II) ou (III) selon l'invention.

Les agents anti-prion selon l'invention sont particulièrement utiles pour l'obtention d'un médicament destiné à prévenir et/ou à traiter les maladies neurodégénératives, en particulier de type à agrégation de protéines, telle que les encéphalopathies spongiformes, les maladies d'Alzheimer (tau), Parkinson (synucléine α) et de Huntington (huntingtine)... Ces médicaments peuvent être à visée humaine ou vétérinaire, en particulier pour des animaux domestiques (vaches, moutons, ...) ou sauvages (lynx, cervidés tels que biches, élans, ...)

D'autres caractéristiques et avantages de l'invention apparaîtront dans les exemples ci-dessous et en se référant aux figures suivantes:
- la figure 1 se rapporte à la faisabilité du crible,
- la figure 2 illustre le protocole de criblage,
- la figure 3 est relative à l'isolement des Kastellpaolitines, de la phénanthridine et à leur relation structure/activité,
- la figure 4 se rapporte à la détermination de l'activité des dérivées de la phénanthridine,
- la figure 5 présente les résultats des tests de cure liquide,
- la figure 6 se rapporte au crible secondaire basé sur le prion [*URE3*],
- la figure 7 démontre la validation du test avec la chlorpromazine, la quinacrine et le verapamil,
- la figure 8 présente les résultats de l'effet de KP1 sur le prion mammifère dans un modèle *in vitro,* et,
- la figure 9 est relative à une étude structure/activité réalisée sur la molécule de formule générale (II).

### Exemple 1 : Réalisation du crible.

### 1. Matériel et méthodes

Organismes *(Saccharomyces cerevisiae*) et milieux de culture La souche de levure haploïde [*PSI+*] 74-D694 (*Mat a*, *ade1-14, trpl-289, his3-d200, ura3-52, leu2-3, 112*) a été utilisée dans la mise au point de la méthode de criblage. La souche utilisée est dite « *Strong* » car elle présente un phénotype bien marqué lorsque le facteur de terminaison de la traduction Sup35p est sous une forme prion ou agrégée.

Afin d'augmenter la pénétration des inhibiteurs, les inventeurs ont modifié génétiquement cette souche en y introduisant une mutation du gène *ERG6.* Ce gène intervient dans la biosynthèse de l'ergostérol, composant de la paroi cellulaire des levures. La mutation a été réalisée par insertion au niveau du site chromosomique du gène *ERG6* d'une « cassette de délétion » correspondant au gène marqueur *TRP1* flanqué par des séquences en ADN se trouvant en amont et en aval de la phase codante du gène *ERG6.* Cette cassette a été produite par PCR en utilisant le plasmide pFA6a-kanMX6 comme matrice et les oligonucléotides oBM1060 (5') et oBM1061 (3') comme amorces. Les cellules de levure 74-D694 « *Strong »* ayant intégré la cassette de délétion (souche appelée **STRg6**, déposée à la CNCM le 10 octobre 2002 sous le numéro I-2943) sont celles qui se développent sur milieux minimum dépourvu en tryptophane. La mutation *Δerg6::TRP1* a ensuite été vérifiée par PCR en utilisant l'ADN génomique de la souche **STRg6** comme matrice et les oligonucléotides oBM1030 (5') et oBM1063 (3') comme amorces.

Les amorces PCR utilisées présentent les séquences en nucléotides suivantes : oBM1030 5' GGTACCTCGTTCCCGTAC 3' (SEQ ID N°3)
oBM1063 5' CAGTCAGAAATCGAGTTCCA 3' (SEQ ID N°4)

Sauf indication du contraire, les souches de levure sont cultivées à 30°C dans du milieu riche (YPDψ) ou dans du milieu minimum. Lorsque ce n'est pas explicitement spécifié, les pourcentages correspondent à un rapport masse/volume. La forme gélosée est obtenue par ajout d'agar à 2%.
YPDψ : 1% d'extrait de levure (FISHER®), 2% de peptone (GIBCO®) et 2% de glucose ;
Milieu minimum : 0,175% de *yeast nitrogen base without amino acid and* ammonium sulfate (DIFCO®), 0,75% de sulfate d'ammonium et 2% de glucose. Ce milieu est amené à pH 6. Afin de compenser les éventuelles auxotrophies, ce milieu peut être complété, après stérilisation, par ajout d'acides aminés (0,002% de L-Histidine et/ou 0,004% de L-Leucine et/ou 0,003% de L-Tryptophane) ou de bases azotées (0,0025% d'Uracile et/ou 0,008% d'Adénine).

### Méthode de criblage de substances à activité anti-prionesque (« Prion Halo Assay »)

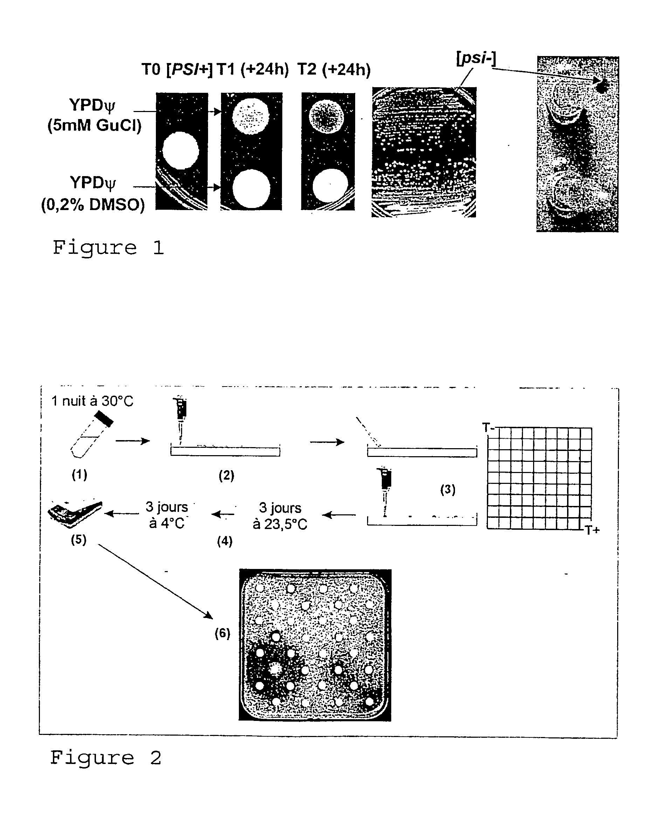

La méthode de criblage élaborée est basée sur le principe de l'antibiogramme. En effet, les composés à tester sont déposés sur un disque en papier filtre stérile, lui-même déposé sur une boîte de milieu YPDψ solide contenant 0,2 mM de chlorure de guanidium préalablement ensemencée avec environ 5.10⁶ cellules de la souche *STRg6* afin de réaliser un tapis de levure. Cette quantité de cellules ensemencées (de 10⁶ à 10⁷) a été optimisée afin que chaque cellule puisse se diviser au moins 6 fois (nombre de génération nécessaire pour avoir un effet de cure efficace avec 3 mM de GuHCl). L'ajout d'une faible quantité de chlorure de guanidium (0,2 mM), dose sub-efficace pour curer les prions chez la levure (la dose efficace étant de l'ordre de 1 à 5 mM) permet d'augmenter la sensibilité du test (voir la partie Résultats). Les boîtes carrées de 12 cm de côté sont ensuite incubées 3 jours à 23,5°C pour permettre l'apparition et la croissance des colonies de levures. Ces boîtes sont ensuite stockées 3 jours à 4°C afin d'accentuer lé coloration rouge présente autour des disques imbibés de substances actives sur la forme prion de la protéine Sup35p. La comparaison avec les témoins négatifs (dépôt du solvant des inhibiteurs testés) et positif (dépôt d'une solution de chlorure de guanidium à 300 mM, provoquant une élimination efficace des protéines Sup35p sous une forme prion) permet de juger de l'efficacité d'un composé. La figure 2 illustre le protocole de la méthode de criblage : (1) Culture de la souche STRg6 ; (2) Dépôt et étalement avec des billes de verre stériles de 3 & 4 mm de diamètre, d'environ 10⁶ cellules en phase exponentielle de croissance sur une boîte de milieu solide YPDψ contenant 0,2 mM de chlorure de guanidium : constitution du "tapis" de cellules ; (3) Dépôt des disques de papier filtre stériles selon un quadrillage permettant l'analyse de 32 composés (témoins inclus) et dépôt de 20 µl maximum de chacun des produits à tester ; (4) Incubation ; (5) Numérisation du résultat obtenu ; (6) Exemple présentant l'isolement d'un composé présentant une forte activité anti-prion.

### Synthèse de 11-aminodibenzo[b,f] [1,4] thiazépines et de la 6-aminophénanthridine

Les 11-aminodibenzo[b,f][1,4]thiazépines, appelés encore Kastellpaolitines, peuvent être préparés en une seule étape. La synthèse de ces produits a déjà été décrite dans la publication de Mettey et al., 1997.

### 2. Résultats

### Principe et faisabilité du crible

Le chlorure de guanidium, le seul produit connu pour curer efficacement les prions chez la levure *Saccharomyces cerevisiae,* a servi non seulement de témoin positif tout au long du criblage, mais aussi pour étudier la faisabilité de la méthode ainsi que pour la mettre au point. Le chlorure de guanidium cure efficacement les différents prions de levure à une dose comprise entre 1 et 5 mM (Fernandez-Bellot et Cullin, 2001). Dans ces conditions, la cure nécessite une présence constante de ce produit pendant six à dix générations en phase exponentielle de croissance compromettant la faisabilité du crible sur boîte tel que les inventeurs souhaitaient le réaliser.

La figure 1 montre la faisabilité du crible.
Les trois panneaux de gauche : une souche [*PSI+*] est cultivée pendant 48H en présence de chlorure de guanidium à 5 mM (avec 0,2% DMSO final) ou, comme contrôle avec seulement 0,2% DMSO final. A T = 0, puis toutes les 24H, une goutte de 10 µL (environ 10⁴ cellules) est déposée sur une boîte de milieu riche. La cure au chlorure de guanidium commence à avoir un effet après 24H de traitement, soit après 6 générations environ (une coloration rosée commence à apparaître). Au bout de 48H, soit après 12 générations environ, la goutte de cellules présente une coloration nettement rouge, signe d'une cure complète des cellules [*PSI*+].
Le panneau du milieu : quelques cellules sont prélevées à T = 48H et sont striées sur un milieu frais. Elles forment presque toutes des colonies rouges dans le cas de la cure au chlorure de guanidium.
Le panneau de droite : ces même cellules sont culottées au fond d'un tube Eppendorf après culture liquide. Dans le cas de la cure au chlorure de guanidium, elles forment un culot rouge.

La première étape a donc consisté à déterminer si le chlorure de guanidium pouvait avoir un effet visualisable sur boîte sur des cellules [PSI+] avec le système de pastilles à antibiogramme. Une fois cette étape réalisée, les inventeurs ont mis au point les conditions optimales de température, de milieu, de densité ainsi que de type cellulaire à utiliser (figure 2). La souche présentant la meilleure sensibilité est la souche STRg6 cultivée à 23,5 °C et en présence de 200 µM de chlorure de guanidium. En effet, l'introduction d'une dose sub-efficace de chlorure de guanidium dans le milieu permet d'augmenter la sensibilité du test.

### Criblage d'une chimiothèque

Des composés (environ 1000) ont été passés au crible en utilisant les conditions optimisées par les inventeurs (figure 2). Sur chaque boîte, 15 µl de DMSO sont déposés sur le filtre en haut à gauche (témoin négatif) et 15 µl d'une solution de chlorure de guanidium en solution à 300 mM dans le DMSO (témoin positif) sont déposés sur le filtre en bas à droite. Le même volume (15 µl) de chacun des produits de la banque (tous en solution à 10 mM dans le DMSO) est déposé sur les filtres restants (trente par grande boîte de Pétri carrée). Un signal positif (visualisation d'un halo rouge autour du disque de papier filtre stérile sur lequel le produit est déposé) a été obtenu pour cinq produits. Ces produits correspondent à quatre molécules d'une même famille, appelées « Kastellpaolitines » par les inventeurs, et à une cinquième bien connue : la phénanthridine.

### Exemple 2 : Identification des Kastellpaolitines et de la phénanthridine.

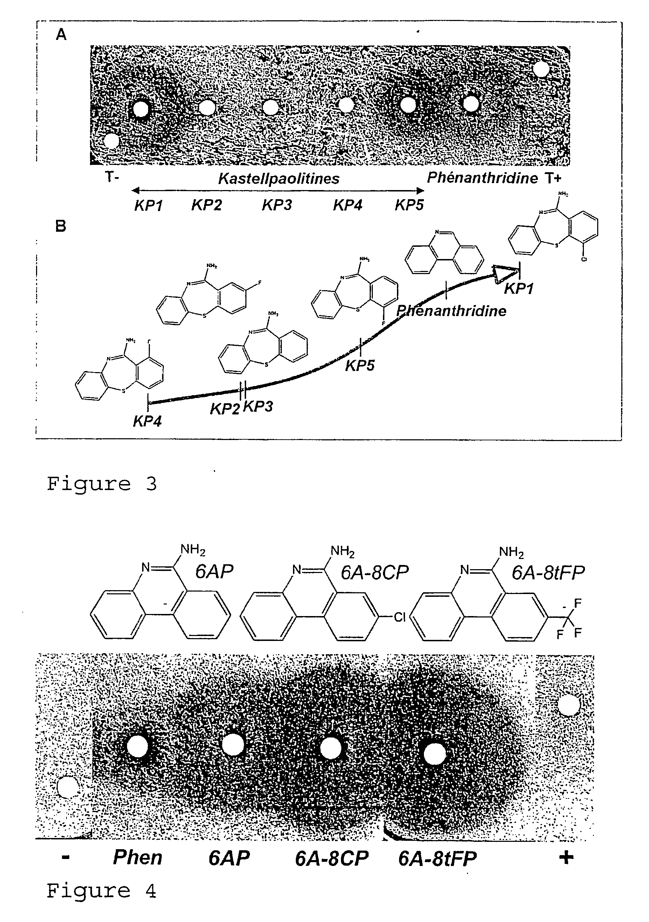

Les structures chimiques des Kastellpaolitines et de la phénanthridine sont présentées dans la figure 3B. Le panneau 3A montre une analyse comparative de la taille des halos rouges obtenus respectivement avec l'ensemble de ces molécules (toutes déposées en quantité équivalentes : 15 µl d'une solution à 10 mM dans le DMSO). Cette expérience permet de comparer l'activité relative de chacun de ces produits. Le plus actif est la Kastellpaolitine 1 (ou KP1) suivi par la phénanthridine.

### Synthèse et test de la 6-aminophénanthridine

Une analyse comparative de la phénanthridine d'une part, et des Kastellpaolitines d'autre part montre plusieurs points communs entre ces deux groupes de molécules (figure 3). Les différentes molécules y sont classées de la moins active à la plus active et leurs formules respectives indiquées. Toutes sont tri-cycliques, le cycle central contenant dans tous les cas un azote en double liaison avec un carbone adjacent. Par contre, chez toutes les Kastellpaolitines, le carbone du cycle central qui est en double liaison avec cette azote porte un groupement amino, ce qui n'est pas le cas pour la phénanthridine. Cette observation a conduit les inventeurs à vouloir tester la 6-aminophénanthridine.

La 6-aminophénanthridine peut être préparé selon le mode opératoire mis au point par Kessar et al, 1969.

La 6-aminophénanthridine a donc été passée au crible selon l'invention, en comparaison avec les Kastellpaolitines 1 (KP1) et 5 (KP5) ainsi qu'avec la phénanthridine. Le résultat est très net : la 6-aminophénanthridine est encore plus active que les Kastellpaolitines et que la phénanthridine.

La figure 4 illustre les résultats de cette comparaison : l'activité de la 6-aminophénanthridine a été déterminée sur boîte et comparée à celle de la phénanthridine. Pour toutes les molécules, la même quantité est déposée (10 µl d'une solution à 10 mM). Dans le cas du témoin positif (chlorure de guanidium), la solution utilisée était à 300 mM.

Par conséquent, en greffant ce groupement amino, caractéristique des Kastellpaolitines sur la phénanthridine, les inventeurs ont augmenté fortement l'activité de cette dernière.

En suivant la même démarche, les inventeurs ont ensuite ajouté un chlore en position 8 dans la 6-aminophénanthridine (6AP) pour produire la 6-amino-8-chlorophénanthridine (6A-8CP). Cette modification a de nouveau augmenté l'activité du composé. Finalement, le chlore en position 8 a été remplacé par un groupe trifluorométhyle pour donner la 6-amino-8-trifluorométhylphénanthridine (6A-8tFP). Comme le montre la figure 4, cette dernière modification a conduit à une augmentation supplémentaire de l'activité et la 6A-8tFP représente actuellement un des composés les plus actifs.

### Exemple 3 : Synergie entre les produits isolés à l'aide du crible et le chlorure de guanidium

Toutes les molécules actives ont été isolées dans un milieu contenant une faible dose de chlorure de guanidium (200 µM / dose efficace = 4 mM). Ce parti pris établi lors de la mise au point du crible répondait au souci d'augmenter la sensibilité (et donc le seuil de détection de la méthode). L'effet des molécules dans des milieux contenant plus (500 µM) de chlorure de guanidium ou n'en contenant pas, a par la suite été observé. La phénanthridine est toujours active sur un milieu sans chlorure de guanidium, mais son activité augmente fortement en fonction de la quantité de chlorure de guanidium (pourtant en dose nettement sub-efficace) dans le milieu. Ce résultat indique une synergie d'action entre le chlorure de guanidium et la phénanthridine. Le même résultat a été obtenu pour toutes les autres molécules isolées par les inventeurs (les Kastellpaolitines, la 6-aminophénanthridine et ses dérivés).

### Exemple 4 : Vérification de la cure en milieu liquide

Les inventeurs ont ensuite voulu déterminer si les halos rouges observés dans le test levure correspondaient bien à de la cure du prion [*PSI*+] et non pas à un artéfact (par exemple ces halos rouges pourraient être dus à une inhibition directe de la chaîne de biosynthèse de l'adénine par ces molécules, ce qui conduirait à une accumulation de l'AIR). Si ces molécules curent efficacement le prion [*PSI*+], un traitement en culture liquide de cellules [PSI+] suivi d'un lavage desdites cellules doit leur permettre de former des colonies rouges sur un milieu gélosé ne contenant plus les molécules. Ces tests ont été réalisés avec la 6-aminophénanthridine sur la souche « strong » sauvage 74-D694.

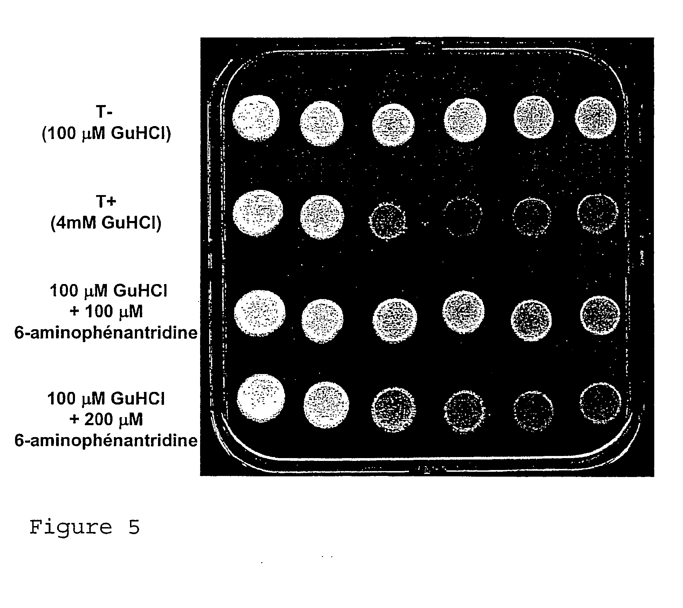

Les conditions de cure en milieu liquide sont les suivantes : une souche *[PSI+]* est cultivée pendant 5 jours en milieu liquide en présence des quantités indiquées des différents produits (voir figure 5). Toutes les 24H, une fraction aliquote est lavée en milieu vierge de tout produit et déposée sur un milieu gélosé solide (lui aussi vierge de tout produit) qui est traité ensuite comme indiqué en figure 2.

Comme montré dans la figure 5, la 6-aminophénanthridine est capable de curer partiellement le prion [PSI+] dans un nombre significatif de cellules. L'efficacité de cure peut être notablement augmentée en rajoutant une dose sub-efficace (100 µM) de chlorure de guanidium dans le milieu de culture. Dans une telle cure liquide, le même effet synergique que celui observé dans le test sur boîte est également retrouvé.

### Exemple 5 : Mise au point et utilisation d'un crible colorimétrique secondaire basé sur l'utilisation de [URE3], un autre prion de levure

Un autre test rapide sur boîte a été réalisé, basé sur un autre prion de levure : *[URE3].* Ce test constitue un crible secondaire qui permet de généraliser l'effet des produits isolés lors du crible primaire à un autre prion de levure. De la sorte, il est possible d'écarter les molécules actives uniquement contre le prion [*PSI*+] et donc moins intéressantes car d'un effet non général.

Pour le prion [*URE3*] la souche haploïde utilisée est CC34 (Mat *a*, *trp1-1, ade2*-*1*, *leu2-3, 112, his3-11, 15, ura2::HIS3).*

La souche **NT34** qui a servi pour le crible secondaire a été construite à partir de CC34, souche dans laquelle la phase codante du gène *DAL5* a été remplacée par celle du gène *ADE2* en utilisant la même méthode que celle utilisée pour la construction de la souche **STRg6**. Pour cela une cassette de délétion correspondant au gène *ADE2* flanqué par des séquences en ADN se trouvant en amont et en aval de la phase codante du gène *DAL5* a été produite par PCR en utilisant de l'ADN génomique de la souche haploïde BY4742 *(Mat α, his3Δ1, leu2Δ0, lys2ΔO, ura3ΔO)* comme matrice et les oligonucléotides :
ACAACAAAACAAGGATAATCAAATAGTGTTCAAGATGGATTCTAGAACAG TTGG (SEQ ID N°5) (5'), et,
TATATTCTTCTCTGATAACAATAATGTCAGTGTATCTCACCACTATTATTACTTGTTTTCTA GATAAGC (SEQ ID N°6) (3') comme amorces.
La mutation *da15::ADE2* a ensuite été vérifiée par PCR en utilisant l'ADN génomique de la souche **NT34** comme matrice et les oligonucléotides :
ATAGTCTCTGCTCATAG (SEQ ID N°7) (5'), et,
GCTTACAGAAATTCTAC (SEQ ID N°8) (3') comme amorces.

La souche **NT34** (*Mat a*, *trp1-1, ade2-1, leu2-3, 112, his3-11, 15, ura2::HIS3, dal5::ADE2)* a été déposée à la CNCM le 10 octobre 2002 sous le numéro I-2942.

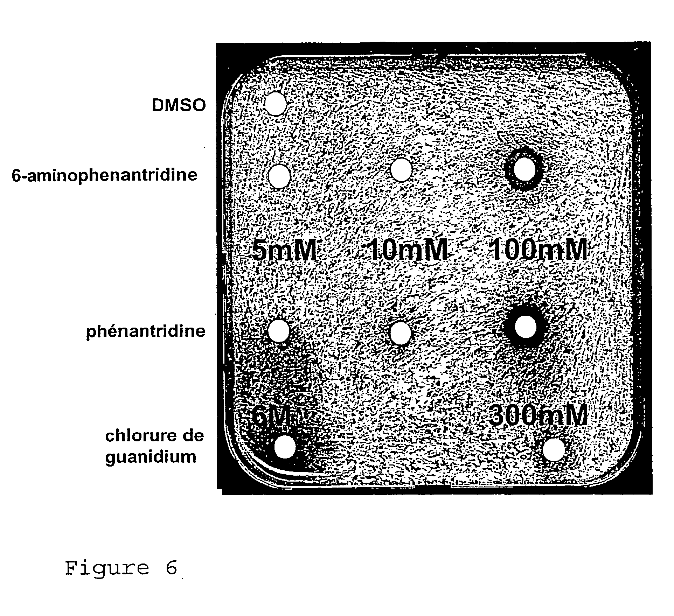

Ce crible est basé sur le même système colorimétrique que le crible primaire. Dans la souche de levure **NT34**, le gène *ADE2* n'est plus sous contrôle de son propre promoteur, mais sous celui du gène *DAL5.* Lorsque la protéine Ure2p est sous forme prion ([*URE3*])*,* la transcription à partir du promoteur du gène *DAL5* est activée donc le gène *ADE2* est exprimé, donc les souches sont blanches et autotrophes pour l'adénine. Lorsque la protéine Ure2p est sous forme normale ([*ure3-0*]), la transcription à partir du promoteur du gène *DAL5* est réprimée donc le gène *ADE2* n'est pas exprimé, donc les souches sont rouges et auxotrophes pour l'adénine. Lorsque la souche NT34 est traitée avec 5mM de chlorure de guanidium pendant une dizaine de générations, elle forme des colonies rouges (comme attendu et comme le ferait la souche [*PSI+*] utilisée pour le criblage primaire). Comme on peut l'observer sur la figure 6, la phénanthridine et la 6-aminophénanthridine provoquent l'apparition d'un halo rouge lorsqu'elles sont déposées sur le petit filtre lui-même déposé sur le tapis de cellules préalablement étalées sur le milieu nutritif gélosé (même procédé que pour le crible primaire, voir figure 2). Ce résultat suggère que ces produits sont également actifs sur le prion [*URE3*]. Il est à noter, toutefois, que ce crible secondaire est nettement moins sensible que le crible primaire. Il est donc très utile pour observer rapidement si l'effet des molécules isolées lors du premier crible est généralisable à d'autres prions de levure mais en aucun cas il ne saurait se substituer au crible primaire.

Afin d'augmenter la perméabilité cellulaire, la séquence codante du gène *ERG6* a également été remplacée par celle du gène *TRP1*. Dans cette souche (SB34), la transcription de *ADE2* dépend donc de l'état de Ure2p : si Ure2p est inactivé par un mécanisme prionique (cellules [*URE3*]), le gène *ADE2* est transcrit activement alors que dans les cellules [*ure3*-*0*], il ne l'est pas. Donc, les cellules [*URE3*] de la souche SB34 formeront des colonies blanches alors que les cellules [ure3-0] formeront des colonies rouges. Du fait que cette souche contient toujours l'allèle *ade2*-*1*, il a été envisagé que cette souche pourrait être [*PSI+*], de sorte que la coloration rouge pourrait être due au curage de [*PSI+*] plutôt que de [*URE3*]*.* Cette possibilité a été exclue en vérifiant par cytoduction et conjugaison que la souche est bien [URE3]. En plus, la séquence codante entière du gène *ade2-1* a été délétée pour donner la souche NT35. Cette souche a également formé des colonies blanches, démontrant de nouveau qu'il s'agit bien de [*URE3*].

La souche SB34 a été construite en remplaçant le gène *ERG6* dans CC34 par amplification PCR du marqueur *TRP1* et en remplaçant la région codante du gène *DAL5* par le gène *ADE2* en utilisant une méthode basée sur la PCR par délétion du gène *ERG6* avec les amorces (5'-ACAACAAAACAAGGATAATCAAATAGTGTAAAAAAA AAAATTCAAGATGGATTCTAGAACAGTTGG-3') (SEQ ID n°9) et 342 (5'-TATATTCTTCTCTGATAACAATAATGTCAGTGTATCTCACCACTATTATTACTTGTTTCTAG ATAAGC-3') (SEQ ID N°10). Ce remplacement de gène a été ensuite confirmé par croissance sur le milieu SD-Ade, en l'absence de croissance sur le milieu USA (comme prévu pour une souche *da15Δ*) et par PCR analytique sur l'ADN génomique. Le phénotype *[URE3]* de cette souche a été vérifié par cytoduction : parmi 30 cytoducteurs, 26 ont été capables de croître sur le milieu USA, montrant qu'il s'agissait de [*URE3*]. La souche NT35 a été construite en remplaçant le gène *ade2*-*1* dans la souche SB34 par le marqueur KanMX amplifié par PCR et en vérifiant le remplacement réussi du gène par PCR analytique sur l'ADN génomique.

### Exemple 6 : Vérification de la cure de [URE3] en milieu liquide

Deux types d'expériences ont été menés afin de vérifier que l'effet observé sur boîte avec la souche NT34 correspond bien à de la cure. Tout d'abord, des cellules dans les zones environnant les filtres ont été récupérées pour le témoin négatif (DMSO), positif (chlorure de guanidium) pour la phénanthridine et pour la 6-aminophénanthridine. Ces cellules ont ensuite été striées sur un milieu frais exempt de toutes ces molécules. Les cellules récupérées autour des filtres forment toutes des colonies rouges, à l'exception de celles récoltées autour du témoin négatif. Ce résultat montre que la coloration rouge observée sur boîte pour la souche NT34 correspond bien à une cure et non à un artéfact lié à une inhibition d'une enzyme de la voie de biosynthèse de l'adénine (dans ce cas, la coloration rouge serait perdue sur un milieu sans inhibiteur). L'effet de cure de la phénanthridine et de la 6-aminophénanthridine a également été vérifié directement sur le prion *[URE3].* Des cellules *[URE3]* de la souche CC34 poussent sur un milieu appelé USA alors que des cellules curées ([*ure3-0*]) sont incapables de pousser sur ce milieu. Les inventeurs ont examiné la capacité de cellules *[URE3]* traitées par 200 µM de chlorure de guanidium (témoin négatif), par 5 mM de chlorure de guanidium (témoin positif) ou par différentes doses de 6-aminophénanthridine (seule ou en combinaison avec 200 µM de chlorure de guanidium) à pousser sur un milieu USA. La 6-aminophénanthridine est capable de curer le prion [URE3] de façon significative et, tout comme pour le prion [*PSI+*], cet effet est accentué par une faible dose de chlorure de guanidium (200 µM). Ces résultats, outre le fait qu'ils valident le crible secondaire avec la souche NT34, suggèrent que l'effet des inhibiteurs mis en évidence par ledit crible devrait être général sur tous les prions de levure.

### Exemple 7 : Validation du crible avec deux molécules actives sur le prion des mammifères PrP : la chlorpromazine et la quinacrine

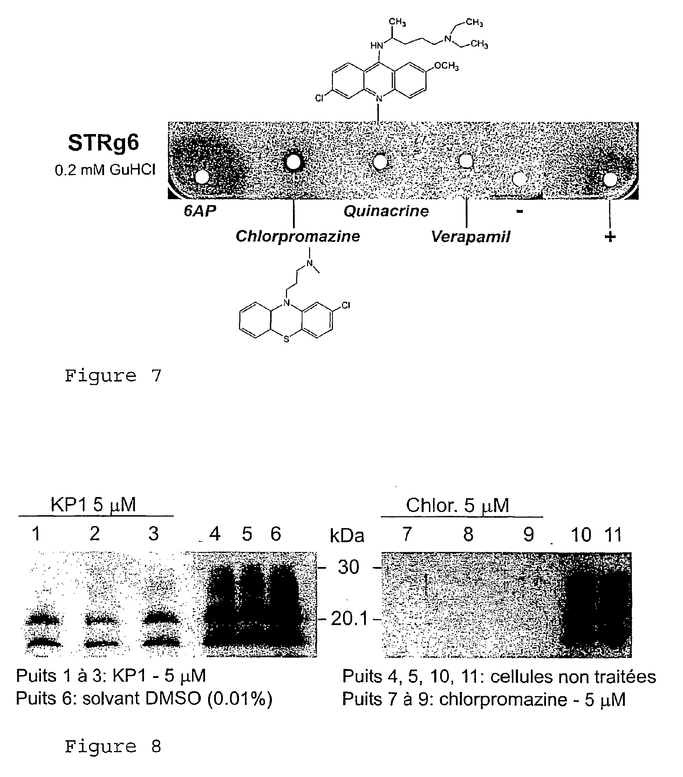

Le laboratoire de Stanley Prusiner, père de l'hypothèse « protéine seule » et prix Nobel en 1997, a isolé un certain nombre de molécules actives sur le prion de mammifère PrP grâce à un système de cellules murines (neuroblastomes) chroniquement infectées par le prion PrP^{SC} (Korth et al., 2001). Ce système, de par sa lourdeur et sa complexité, ne permet pas un criblage massif comme celui mis au point par les inventeurs. Aussi l'approche du groupe de Stanley Prusiner a-t-elle été de tester une à une, parmi des molécules déjà utilisées comme médicaments, celles qui passent la barrière hémato-encéphalique. Certaines molécules, comme notamment la quinacrine (utilisée comme anti-paludéen depuis longtemps) ou la chlorpromazine (un anti-dépresseur) présentent une activité notable dans leur système. De façon à valider le crible, les inventeurs ont donc testé la chlorpromazine et la quinacrine dans leur système levure. Comme montré dans la figure 7, ces deux molécules présentent une certaine activité contre le prion [*PSI+*]*.* Il faut toutefois noter que leurs activités sont nettement inférieures à celle de la 6-aminophénanthridine. On peut également relever que la chlorpromazine et la quinacrine, tout comme l'ensemble des molécules mis en évidence par l'invention, présentent une forte synergie d'action avec le chlorure de guanidium (Sur la figure 7, le milieu utilisé contient 200 µM de chlorure de guanidium). Ce dernier résultat suggère que ces deux molécules agissent sur la même voie biochimique que les molécules isolées selon l'invention.

Par ailleurs, il est intéressant de noter que la quinacrine, dont l'activité est environ dix fois supérieure à celle de la chlopromazine dans le test du Pr. Prusiner, présente également une activité nettement supérieure à celle-ci dans le crible mis au point par les inventeurs. En outre, tout comme dans le test du Pr. Prusiner, la chlorpromazine et la quinacrine nécessitent un traitement prolongé (au moins 6 jours dans le cas du test du Pr. Prusiner, au moins deux à trois jours dans le cas du crible selon l'invention) avant de déceler une activité.

De plus, les inventeurs ont déterminé l'activité, dans le test selon l'invention, d'autres molécules isolées grâce au test basé sur les neuroblastomes de souris, mis au point par le Pr. Prusiner. Il a été trouvé une bonne corrélation entre les résultats obtenus dans les deux systèmes : l'acépromazine qui s'est révélée être légèrement active dans le système mammifère a également présenté une faible activité dans le test selon l'invention et les molécules inactives dans l'analyse sur les mammifères comme la carbamazépine, l'imipramine, l'halopéridol, le chloroprothixène ou le bleu de méthylène ont été également inactives dans le test.

La quinacrine a été également décrite comme inhibiteur de la résistance multiple aux médicaments (MDR). Pour tester si son effet anti-prionique pourrait concerner ce mécanisme (qui est compatible avec l'effet synergique du GuHCl), nous avons évalué l'effet curatif putatif d'un inhibiteur général de MDR efficace, le vérapamil. Comme le montre la figure 7, bien qu'une concentration forte de ce médicament ait été utilisée, concentration proche de la toxicité, aucun effet curatif n'a pu être décelé.

Toutes ces corrélations entre l'activité de la quinacrine et de la chlorpromazine selon le test ou le crible utilisé permettent de valider l'utilisation de la méthode selon l'invention pour réaliser des criblages haut débit en vue d'isoler des molécules susceptibles de constituer des médicaments efficaces (sur les mammifères et en particulier sur l'homme) contre des maladies neurodégénératives impliquant des agrégats protéiques, de type encéphalopathies spongiformes, maladies d'Alzheimer, de Huntington...

### Exemple 8 : Analyse de l'inhibition de la PrP^{SC} dans les cellules ScN2a-22L

Des cellules de neuroblastome de souris infectées par le prion de la tremblante du mouton (ScN2a-22L) ont été utilisées. Les cellules ont été cultivées dans des flacons de 25 cm² en présence ou absence des composés pendant plusieurs jours. Ensuite, les protéines ont été extraites des cellules ScN2a-22L par lyse cellulaire dans 500 µl de tampon de lyse (50 mM de Tris HCl pH 7,5 ; 150 mM de NaCl, 0,5 % de désoxycholate de sodium ; 0,5 % de Triton X100). Après normalisation des protéines avec le kit *Uptima Interchim,* les quantités ajustées de lysats cellulaires ont été digérées par la protéinase K à 20 µg/ml *(Eurobio)* pendant 40 min à 37°C. Les lysats ont été centrifugés ensuite pendant 90 min à 20 000 x g et le culot a été resuspendu dans 25 µl de tampon dénaturant (1 X Tris-Glycine ; 4 % de SDS, 2 % de β-mercaptoéthanol ; 5 % de sucrose et du bleu de bromophénol) et chauffé pendant 5 min à 100°C avant analyse par Western blot selon le protocole standard en utilisant l'anticorps monoclonal de souris anti-PrP SAF83 (fourni par *SPI-BIO,* Massy-Palaiseau, France). Les pourcentages d'inhibition de la formation de PrP^{SC} résistant à la protéinase K ont été calculés en utilisant le *NIH Image J :* l'inhibition de l'accumulation de la PrP^{SC} a été de 96 % pour la chlorpromazine (Chlor.) et 70 % +/- 6 % pour la KP1.

Deux des composés sélectionnés (KP1 et 6AP) ont été testés dans ce système mammifère. Comme le montre la figure 8, la KP1 a été capable d'induire une diminution significative de l'accumulation de prion mammifère à une dose similaire à celle utilisée pour la chlorpromazine (5 *µ*M). Au bout de 7 jours de traitement, 70 % de la PrP^{sc} résistant à la protéinase K ont disparu (puits 1 à 3) comparé aux cellules non traitées (puits 4 et 5). Cet effet significatif a été probablement sous-estimé puisque les cellules traitées avec le solvant des composés seul (DMSO 0,01 %) ont montré une hausse significative et reproductible en PrP^{SC} résistant à la protéinase K (puits 6). Le même effet sur l'élimination de la PrP^{SC} a été obtenu avec la 6AP à 2 et 4 µM.

Ces résultats valident donc l'utilisation du test de criblage selon l'invention basé sur la levure pour isoler les composés anti-prions puisque la quinacrine et la chlorpromazine ont été détectées en utilisant cette analyse et que la KP1 et 6AP ont été également efficaces pour favoriser l'élimination du prion mammifère *in vitro.*

### Exemple 9 : Etude structure/activité

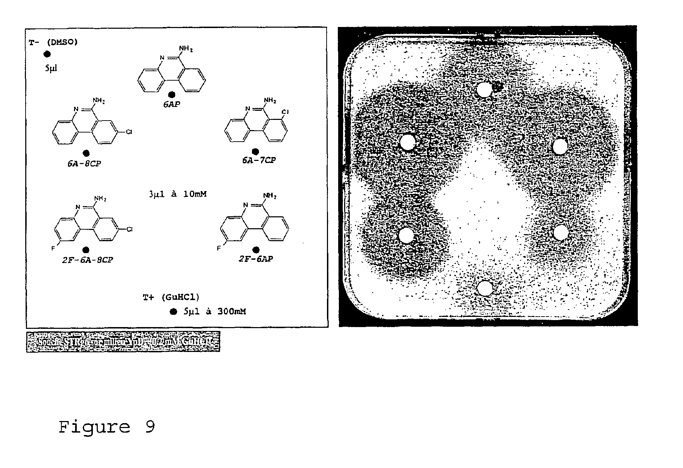

Dans le but d'étudier les différentes positions de substitution des molécules anti-prions isolées, les inventeurs ont réalisé une étude structure/activité sur la molécule de 6-aminophénanthridine. Les molécules de 2-fluoro-6-aminophénanthridine (2F-6AP), 2-fluoro-6-amino-8-chloro-phénanthridine (2f-6A-8ClP) et 6-amino-7-chlorophénanthridine (6A-7ClP) ont ainsi été obtenues par synthèse chimique et leur activité anti-prion a été déterminée en utilisant le test selon l'invention. Les résultats obtenus sont repris par la figure 9. Les diamètres des halos rouges obtenus étant proportionnels à l'activité anti-prions des molécules déposées, les résultats indiquent que la présence d'un substituant de type halogène au niveau des positions 7 ou 8 accroît l'activité anti-prion des molécules de formules (II) tandis que le même type de substituant en position 2 tend à la diminuer.

### Références bibliographiques

- **Fernandez-Bellot et al.,**: "*The protein-only theory and the yeast Saccharomyces cerevisiae: the prions and the propagons",* CMLS, 2001, **58**:1857-1878.
- **Korth C. et al.,**: *"Acridine and phenothiazine derivatives as pharmacotherapeutics for prion disease"*, PNAS, 2001, **98**(17):9836-9841.
- **Mettey Y. et al.,**: "*Synthesis of 11-Aminodibenzo [b*, *f] [1, 4] thiazepines and Fluoro derivatives",* J. Heterocyclic Chem., 1997, **34**:465-467.
- **Kessar S.V. et al.,**: Tetrahedron Letters, 1969, 1151.

### SEQUENCE LISTING

<110> CENTRE NATIONAL DE LA RECHERCHE SCIENTIFIQUE (CNRS)
   UNIVERSITE VICTOR SEGALEN
   UNIVERSITE DE POITIERS
<120> "Criblage de molécules à activité anti-prion : kits, mét hodes et molécules criblées"
<130> CP/BT/61032 PCT
<140> PCT/FR03/03101
   <141> 2003-10-20
<150> FR 03 08 289
   <151> 2003-07-07
<150> FR 02 13 022
   <151> 2002-10-18
<160> 10
<170> PatentIn version 3.1
<210> 1
   <211> 84
   <212> DNA
   <213> Artificial sequence
<220>
   <223> primer
<400> 1
<210> 2
   <211> 84
   <212> DNA
   <213> Artificial sequence
<220>
   <223> primer
<400> 2
<210> 3
   <211> 18
   <212> DNA
   <213> Artificial sequence
<220>
   <223> primer
<400> 3
   ggtacctcgt tcccgtac
   18
<210> 4
   <211> 20
   <212> DNA
   <213> Artificial sequence
<220>
   <223> primer
<400> 4
   cagtcagaaa tcgagttcca
   20
<210> 5
   <211> 66
   <212> DNA
   <213> artificial sequence
<220>
   <223> primer
<400> 5
<210> 6
   <211> 69
   <212> DNA
   <213> Artificial sequence
<220>
   <223> primer
<400> 6
<210> 7
   <211> 17
   <212> DNA
   <213> Artificial sequence
<220>
   <223> primer
<400> 7
   atagtctctg ctcatag
   17
<210> 8
   <211> 17
   <212> DNA
   <213> Artificial sequence
<220>
   <223> primer
<400> 8
   gcttacagaa attctac 17
<210> 9
   <211> 66
   <212> DNA
   <213> Artificial sequence
<220>
   <223> primer
<400> 9
<210> 10
   <211> 68
   <212> DNA
   <213> Artificial sequence
<220>
   <223> primer
<400> 10

## Revendications

1. Kit de criblage de molécules à activité anti-prion, **caractérisé en ce qu'**il comporte en combinaison une levure de phénotype [*PSI+*], un antibiogramme et un agent de curage des prions à des doses sub-efficaces, ladite levure présentant l'allèle *ade1-14* du gène ADE1 ainsi qu'un gène *ERG6* inactivé.

2. Kit selon la revendication 1, **caractérisé en ce que** la levure est *Saccharomyces cerevisiae.*

3. Kit selon la revendication 1 ou 2, **caractérisé en ce que** l'agent de curage des prions est le chlorure de guanidium.

4. Méthode de criblage de molécules à activité anti-prion, **caractérisée en ce qu'**elle met en oeuvre une levure de phénotype [PSI+] présentant l'allèle *ade1-14* du gène ADE1 ainsi qu'un gène *ERG6* inactivé et comporte les étapes suivantes :
a. réalisation d'un tapis de cellules in vitro sur un milieu complémenté d'une dose sub-efficace d'un agent de curage des prions,
b. dépôt des composés à tester selon la méthode de l'antibiogramme,
c. incubation pendant environ 2-4 jours à environ 20-25°C, et,
d. analyse de la coloration des colonies cellulaires.

5. Méthode de criblage selon la revendication 4, **caractérisée en ce que** la levure est *Saccharomyces cerevisiae.*

6. Méthode de criblage selon l'une quelconque des revendications 4 ou 5, **caractérisée en ce que** l'agent de curage de l'étape a. est le chlorure de guanidium.

7. Méthode de criblage selon l'une quelconque des revendications 4 à 6, **caractérisée en ce qu'**elle comporte en outre les étapes suivantes :
e. incubation pendant environ 2-4 jours à environ 2-6°C, et/ou,
f. réalisation d'un test de criblage secondaire.

8. Méthode de criblage selon la revendication 7, **caractérisée en ce que** le test de criblage secondaire comporte les étapes suivantes :
- construction d'une souche de levure dans laquelle le gène *ADE2* est sous le contrôle du promoteur du gène *DAL5*
- réalisation des étapes a. à e. des méthodes selon les revendications 4 et 7.

9. Composé de formule (II) dans laquelle : 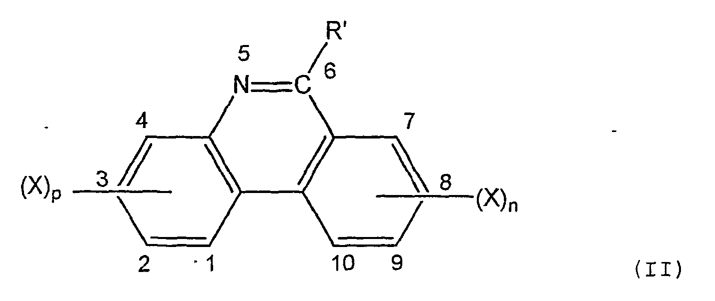 R' représente un groupement H, NH₂, NH- (CH₂)₃-N (CH₃)₂, NH-CH (CH₃) - (CH₂) ₃-N (CH₂-CH₃)₂,
X représente F, Cl, CF₃,
p et n, identiques ou différents, égalent 0, 1 ou 2,
pour une utilisation comme médicament.

10. Composé selon la revendication 9, de formule (II) dans laquelle : 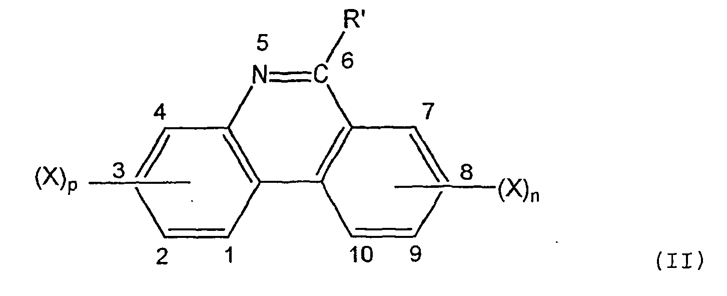 R' représente un groupement NH₂,
X représente F, Cl, CF₃,
p et n, identiques ou différents, égalent 0, 1 ou 2,
pour une utilisation comme médicament.

11. Utilisation du composé de formule (I) 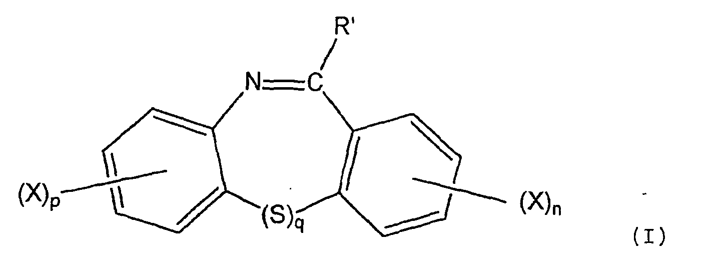 dans laquelle R' est un groupement H, NH₂, NHR², où R² est une chaîne alkyle ou alkylaminoalkyle de 1 à 10 carbones, ramifiée ou non,
X représente F, Cl, Br, I, CF₃, SCH₃, OCH₃, OH, NO₂, COCH₃, CONH₂, COOH, COOR³, où R³ est un groupement alkyl de 1 à 4 carbones,
p et n, identiques ou différents, égalent 0, 1 ou 2,
q égale 0 ou 1,
pour l'obtention d'un médicament destiné à traiter les maladies neurodégénératives impliquant des agrégats protéiques.

12. Utilisation du composé de formule (III) dans laquelle : 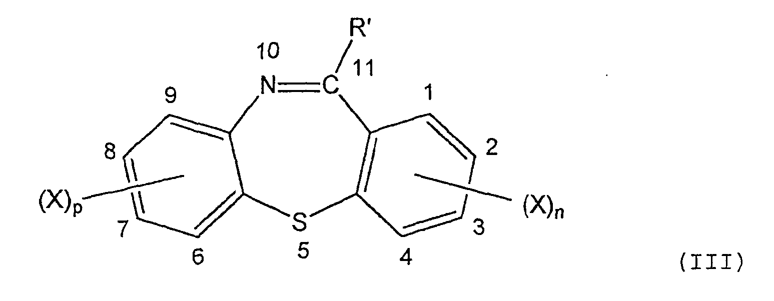 R' représente un groupement H, NH₂, NH-(CH₂)₃-N(CH₃)₂, NH-CH (CH₃)-(CH₂)₃-N(CH₂-CH₃)₂,
X représente F, Cl, CF₃,
p et n, identiques ou différents, égalent 0, 1 ou 2,
pour l'obtention d'un médicament destiné à traiter les maladies neurodégénératives impliquant des agrégats protéiques.

13. Utilisation du composé de formule (II) dans laquelle : 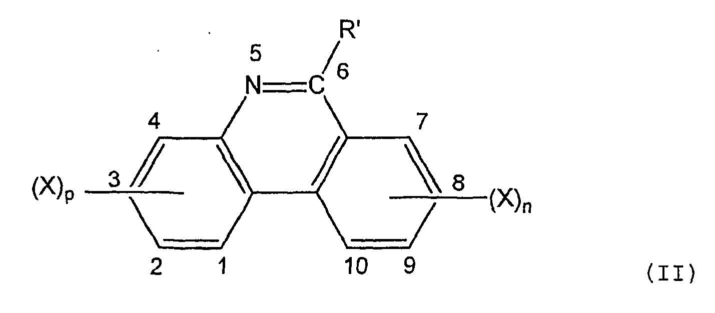 R' représente un groupement H, NH₂, NH- (CH₂) ₃-N (CH₃) ₂, NH-CH (CH₃) - (CH₂) ₃-N(CH₂-CH₃)₂,
X représente F, Cl, CF₃,
p et n, identiques ou différents, égalent 0, 1 ou 2, pour l'obtention d'un médicament destiné à traiter les maladies neurodégénératives impliquant des agrégats protéiques.

14. Utilisation selon la revendication 13, du composé de formule (II) dans laquelle : 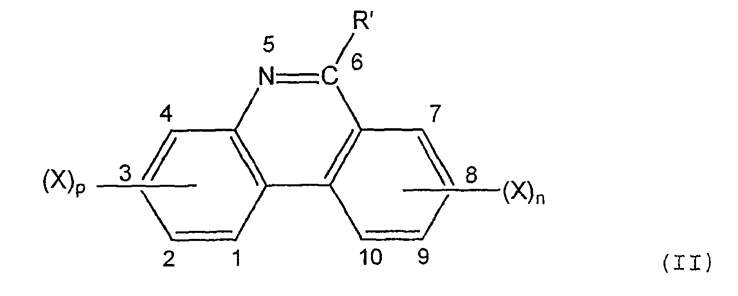 R' représente un groupement NH₂,
X représente F, Cl, CF₃,
p et n, identiques ou différents, égalent 0, 1 ou 2, pour l'obtention d'un médicament destiné à traiter les maladies neurodégénératives impliquant des agrégats protéiques.

15. Utilisation selon les revendications 11 à 15, **caractérisée en ce que** les maladies neurodégénératives sont les encéphalopathies spongiformes, les maladies d'Alzheimer et de Huntington.

16. Composition pharmaceutique comprenant une quantité thérapeutiquement efficace d'au au moins un composé de formule (II) dans laquelle : 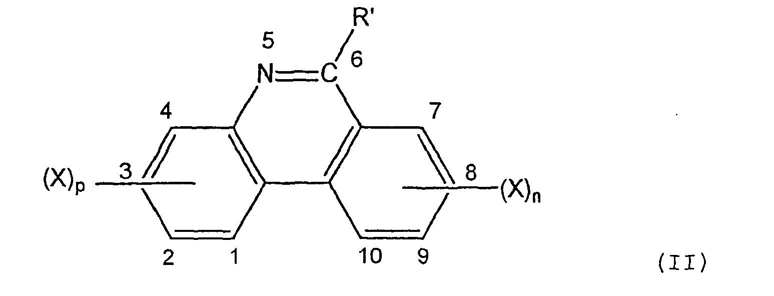 R' représente un groupement H, NH₂, NH- (CH₂)₃-N (CH₃)₂,
NH-CH (CH₃) - (CH₂) ₃-N (CH₂-CH₃)₂,
X représente F, Cl, CF₃,
p et n, identiques ou différents, égalent 0, 1 ou 2, en association avec au moins un véhicule pharmaceutiquement acceptable.

17. Composition pharmaceutique, selon la revendication 16, comprenant une quantité thérapeutiquement efficace d'au moins un composé de formule (II) dans laquelle : 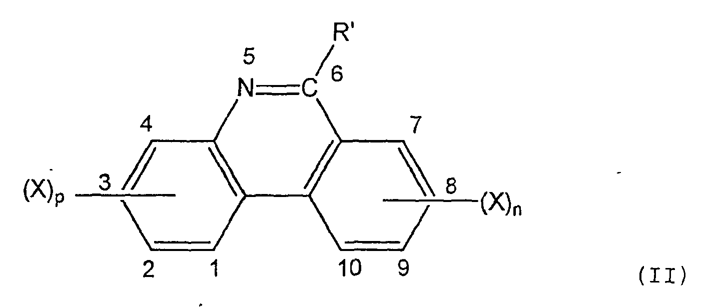 R' représente un groupement NH₂,
X représente F, Cl, CF₃,
p et n, identiques ou différents, égalent 0, 1 ou 2, en association avec au moins un véhicule pharmaceutiquement acceptable.

## Claims

1. Kit for screening molecules with an anti-prion activity, **characterized in that** it comprises in combination a yeast of phenotype [*PSI+*], an antibiogram and a prion curing agent in sub-effective doses, said yeast having the *ade1-14* allele of the *ADE1* gene as well as an inactivated *ERG6* gene.

2. Kit according to claim 1, **characterized in that** the yeast is *Saccharomyces cerevisiae.*

3. Kit according to claim 1 or 2, **characterized in that** the prion curing agent is guanidium chloride.

4. Method for screening molecules with anti-prion activity, **characterized in that** it uses a [PSI+] phenotype yeast having the *ade1-14* allele of the *ADE1* gene as well as an inactivated *ERG6* gene and comprises the following stages:
a. production of a lawn of cells *in* vitro on a medium complemented with a sub-effective dose of a prion curing agent,
b. deposition of the compounds to be tested according to the antibiogram method,
c. incubation for approximately 2-4 days at approximately 20-25°C, and,
d. analysis of the staining of the cell colonies.

5. Screening method according to claim 4, **characterized in that** the yeast is *Saccharomyces cerevisiae.*

6. Screening method according to any one of claims 4 or 5, **characterized in that** the curing agent of Stage a. is guanidium chloride.

7. Screening method according to any one of claims 4 to 6, **characterized in that** it moreover comprises the following stages:
e. incubation for approximately 2-4 days at approximately 2-6°C, and/or,
f. carrying out a secondary screening test.

8. Screening method according to claim 7, **characterized in that** the secondary screening test comprises the following stages:
- construction of a strain of yeast in which the *ADE2* gene is under the control of the *DAL5* gene promoter
- carrying out Stages a. to e. of the methods according to claims 4 and 7.

9. Compound of formula (II) in which: 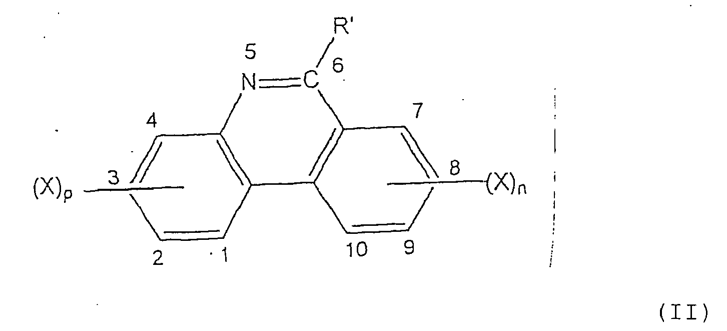 R' represents an H, NH₂, NH- (CH₂) ₃-N (CH₃) ₂, NH-CH (CH₃) - (CH₂) ₃-N (CH₂-CH₃) ₂ group,
X represents F, Cl, CF₃,
p and n, identical or different, are equal to 0, 1 or 2
for use as a medicament.

10. Compound according to claim 9, of formula (II) in which: 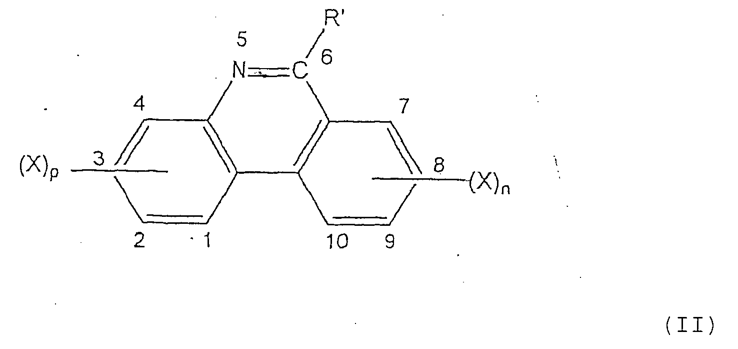 R' represents an NH₂ group,
X represents F, Cl, CF₃,
p and n, identical or different, are equal to 0, 1 or 2,
for use as a medicament.

11. Use of the compound of formula (I) 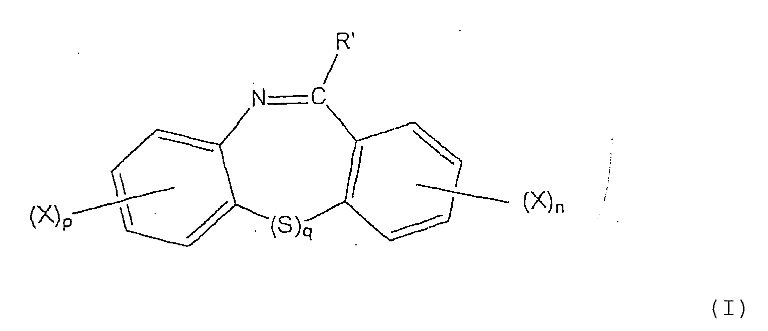 in which R' is an H, NH₂, NHR² group, where R² is an alkyl or alkylaminoalkyl chain with 1 to 10 carbon atoms, branched or unbranched,
X represents F, Cl, Br, I, CF₃, SCH₃, OCH₃, OH, NO₂, COCH₃, CONH₂, COOH, COOR³, where R³ is an alkyl group with 1 to 4 carbon atoms,
p and n, identical or different, are equal to 0, 1 or 2,
q is equal to 0 or 1,
in order to obtain a medicament intended for treating neurodegenerative diseases involving protein aggregates.

12. Use of the compound of formula (III) in which: 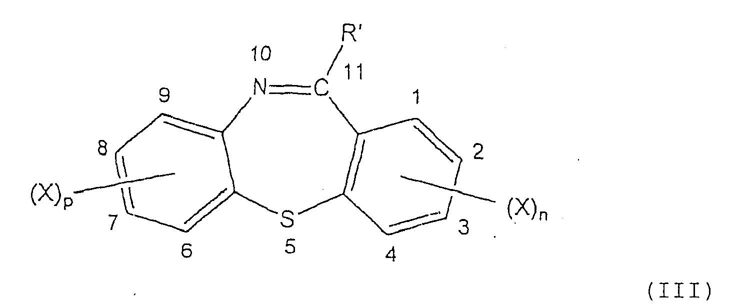 R' represents an H, NH₂, NH- (CH₂)₃-N (CH₃)₂, NH-CH (CH₃) - (CH₂)₃-N (CH₂-CH₃)₂ group,
X represents F, Cl, CF₃,
p and n, identical or different, are equal to 0, 1 or 2.
in order to obtain a medicament intended for treating neurodegenerative diseases involving protein aggregates.

13. Use of the compound of formula (II) in which: 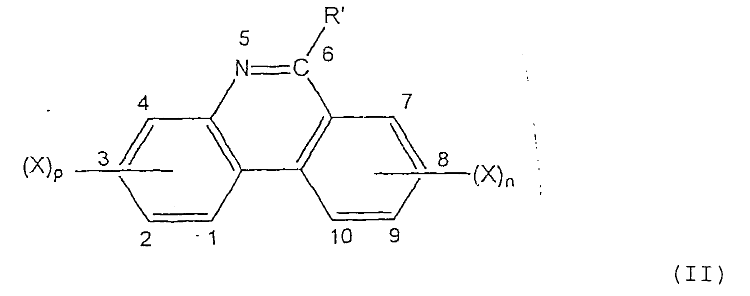 R' represents an H, NH₂, NH- (CH₂)₃-N (CH₃)₂, NH-CH (CH₃) - (CH₂) ₃-N (CH₂-CH₃) ₂ group,
X represents F, Cl, CF₃,
p and n, identical or different, are equal to 0, 1 or 2,
in order to obtain a medicament intended for treating neurodegenerative diseases involving protein aggregates.

14. Use according to claim 13, of the compound of formula (II) in which: 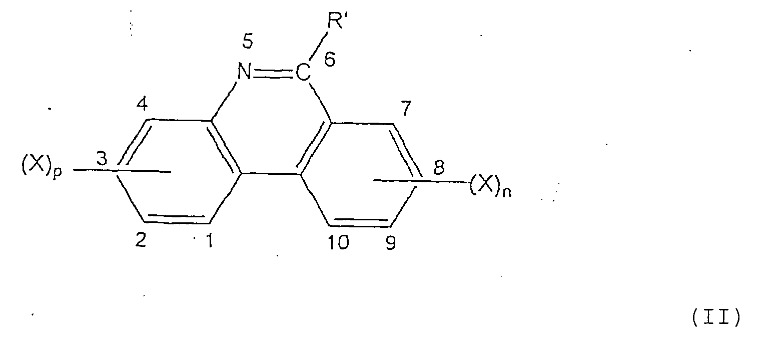 R' represents an NH₂ group,
X represents F, Cl, CF₃,
p and n, identical or different, are equal to 0, 1 or 2,
in order to obtain a medicament intended for treating neurodegenerative diseases involving protein aggregates.

15. Use according to claims 11 to 15, **characterized in that** the neurodegenerative diseases are the spongiform encephalopathies, Alzheimer's disease and Huntington's disease.

16. Pharmaceutical composition comprising a therapeutically effective quantity of at least one compound of formula (II) in which: 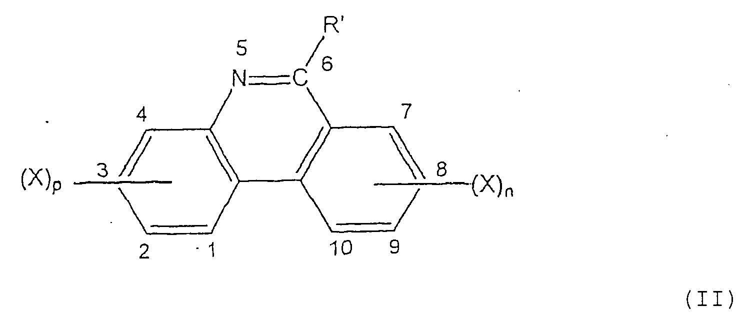 R' represents an H, NH₂, NH- (CH₂)₃-N (CH₃)₂, NH-CH (CH₃) - (CH₂)₃-N (CH₂-CH₃)₂ group,
X represents F, Cl, CF₃,
p and n, identical or different, are equal to 0, 1 or 2.
in combination with at least one pharmaceutically acceptable vehicle.

17. Pharmaceutical composition according to claim 16 comprising a therapeutically effective quantity of at least one compound of formula (II) in which: 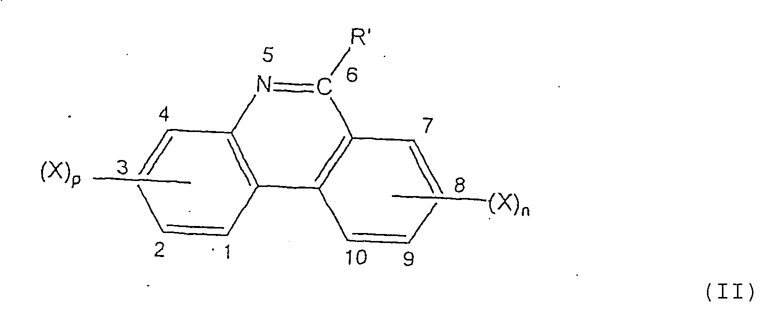 R' represents an NH₂ group,
X represents F, Cl, CF₃,
p and n, identical or different, are equal to 0, 1 or 2,
in combination with at least one pharmaceutically acceptable vehicle.

## Patentansprüche

1. Kit zum Screening von Molekülen auf Antiprion-Aktivität, **dadurch gekennzeichnet, dass** er in Kombination eine Hefe des Phänotyps [PSI+], ein Antibiogramm und ein Prionen-Reinigungsmittel in subeffizienten Dosen umfasst, wobei die Hefe das Allel ade1-14 des Gens ADE1 sowie ein inaktiviertes ERG6-Gen aufweist.

2. Kit gemäß Anspruch 1, **dadurch gekennzeichnet, dass** es sich bei der Hefe um *Saccharomyces cerevisiae* handelt.

3. Kit gemäß Anspruch 1 oder 2, **dadurch gekennzeichnet, dass** es sich bei dem Prionen-Reinigungsmittel um Guanidiniumchlorid handelt.

4. Verfahren zum Screening von Molekülen auf Antiprion-Aktivität, **dadurch gekennzeichnet, dass** eine Hefe des Phänotyps [PSI+], die das Allel ade1-14 des Gens ADE1 sowie ein inaktiviertes ERG6-Gen aufweist, eingesetzt wird und das Verfahren die folgenden Schritte umfasst:
a. Herstellung eines Zellteppichs in vitro auf einem Medium, das mit einer subeffizienten Dosis eines Prionen-Reinigungsmittels versetzt ist;
b. Aufbringen der zu testenden Verbindungen gemäß der Methode des Antibiogramms;
c. Inkubation während ungefähr 2-4 Tagen bei ungefähr 20-25 °C; und
d. Analyse der Färbung der Zellkolonien.

5. Verfahren zum Screening gemäß Anspruch 4, **dadurch gekennzeichnet, dass** es sich bei der Hefe um *Saccharomyces cerevisiae* handelt.

6. Verfahren zum Screening gemäß einem der Ansprüche 4 oder 5, **dadurch gekennzeichnet, dass** es sich bei dem Reinigungsmittel in Schritt a. um Guanidiniumchlorid handelt.

7. Verfahren zum Screening gemäß einem der Ansprüche 4 bis 6, **dadurch gekennzeichnet, dass** es weiterhin die folgenden Schritte umfasst:
e. Inkubation während ungefähr 2-4 Tagen bei ungefähr 2-6°C; und/oder
f. Durchführung eines sekundären Screening-Tests.

8. Verfahren zum Screening gemäß Anspruch 7, **dadurch gekennzeichnet, dass** der sekundäre Screening-Test die folgenden Schritte umfasst:
- Aufbau eines Hefestamms, bei dem das Gen ADE2 unter der Kontrolle des Promotors des Gens DAL5 steht;
- Durchführung der Schritte a. bis e. der Verfahren gemäß den Ansprüchen 4 und 7.

9. Verbindung der Formel (II) 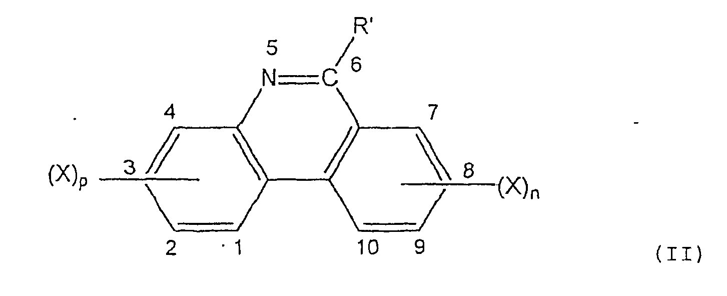 wobei
R' für eine Gruppe H, NH₂, NH-(CH₂)₃-N(CH₃)₂, NH-CH(CH₃)-(CH₂)₃-N(CH₂-CH₃)₂ steht;
X für F, Cl, CF₃ steht;
p und n gleich oder verschieden sind und gleich 0, 1 oder 2 sind;
zur Verwendung als Medikament.

10. Verbindung gemäß Anspruch 9 der Formel (II) 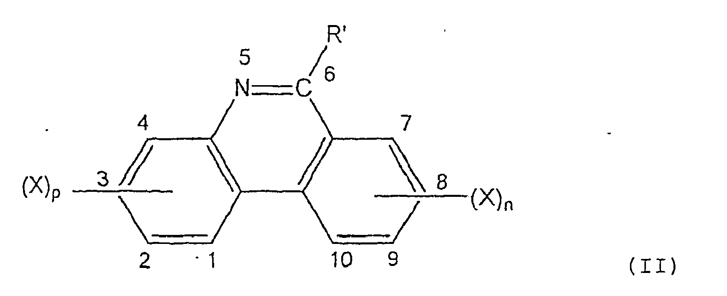 wobei
R' für eine Gruppe NH₂ steht;
X für F, Cl, CF₃ steht;
p und n gleich oder verschieden sind und gleich 0, 1 oder 2 sind;
zur Verwendung als Medikament.

11. Verwendung einer Verbindung der Formel (I) 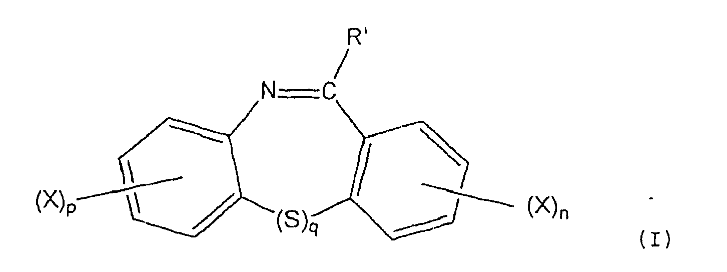 wobei
R' für eine Gruppe H, NH₂, NHR² steht, wobei R² eine verzweigte oder unverzweigte Alkyl- oder Alkylaminoalkylkette mit 1 bis 10 Kohlenstoffatomen ist;
X für F, Cl, Br, I, CF₃, SCH₃, OCH₃, OH, NO₂, COCH₃, CONH₂, COOH, COOR³ steht, wobei R³ eine Alkylgruppe mit 1 bis 4 Kohlenstoffatomen ist;
p und n gleich oder verschieden sind und gleich 0, 1 oder 2 sind;
q gleich 0 oder 1 ist;
zur Gewinnung eines Medikaments zur Behandlung von neurodegenerativen Krankheiten, die Proteinaggregate beinhalten.

12. Verwendung einer Verbindung der Formel (III) 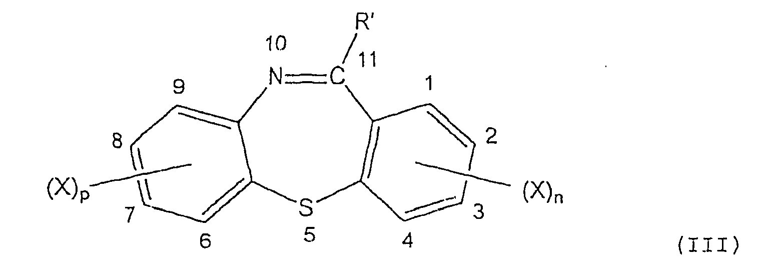 wobei
R' für eine Gruppe H, NH₂, NH-(CH₂)₃-N(CH₃)₂, NH-CH(CH₃)-(CH₂)₃-N(CH₂-CH₃)₂ steht;
X für F, Cl, CF₃ steht;
p und n gleich oder verschieden sind und gleich 0, 1 oder 2 sind;
zur Gewinnung eines Medikaments zur Behandlung von neurodegenerativen Krankheiten, die Proteinaggregate beinhalten.

13. Verwendung einer Verbindung der Formel (II) 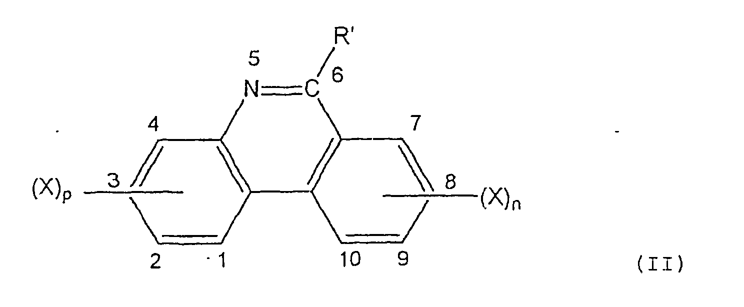 wobei
R' für eine Gruppe H, NH₂, NH-(CH₂)₃-N(CH₃)₂, NH-CH(CH₃)-(CH₂)₃-N(CH₂-CH₃)₂ steht;
X für F, Cl, CF₃ steht;
p und n gleich oder verschieden sind und gleich 0, 1 oder 2 sind;
zur Gewinnung eines Medikaments zur Behandlung von neurodegenerativen Krankheiten, die Proteinaggregate beinhalten.

14. Verwendung gemäß Anspruch 13 der Verbindung der Formel (II) 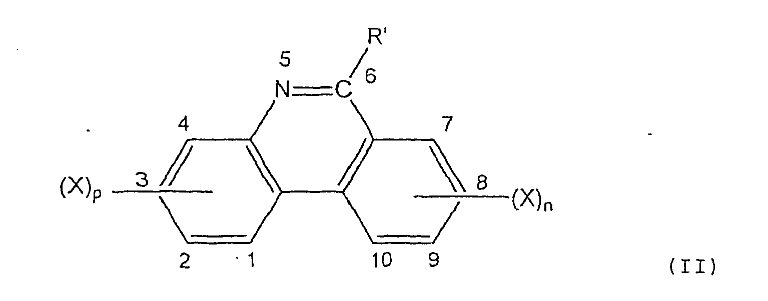 wobei
R' für eine Gruppe NH₂ steht;
X für F, Cl, CF₃ steht;
p und n gleich oder verschieden sind und gleich 0, 1 oder 2 sind;
zur Gewinnung eines Medikaments zur Behandlung von neurodegenerativen Krankheiten, die Proteinaggregate beinhalten.

15. Verwendung gemäß den Ansprüchen 11 bis 14, **dadurch gekennzeichnet, dass** es sich bei den neurodegenerativen Krankheiten um spongiforme Enzephalopathien, Alzheimer-Krankheit und Chorea Huntington handelt.

16. Pharmazeutische Zusammensetzung, die eine therapeutisch wirksame Menge wenigstens einer Verbindung der Formel (II) umfasst: 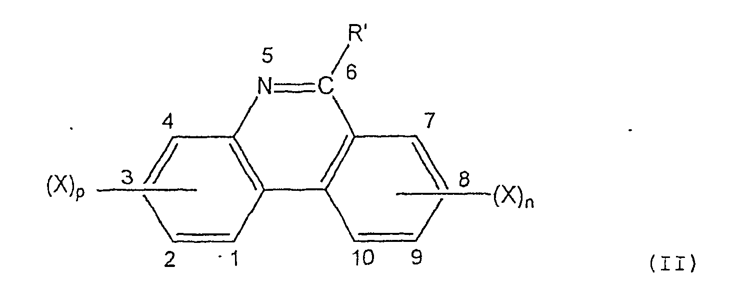 wobei
R' für eine Gruppe H, NH₂, NH-(CH₂)₃-N(CH₃)₂, NH-CH(CH₃)-(CH₂)₃-N(CH₂-CH₃)₂ steht;
X für F, Cl, CF₃ steht;
p und n gleich oder verschieden sind und gleich 0, 1 oder 2 sind;
in Verbindung mit wenigstens einem pharmazeutisch annehmbaren Träger.

17. Pharmazeutische Zusammensetzung gemäß Anspruch 16, die eine therapeutisch wirksame Menge wenigstens einer Verbindung der Formel (II) umfasst: 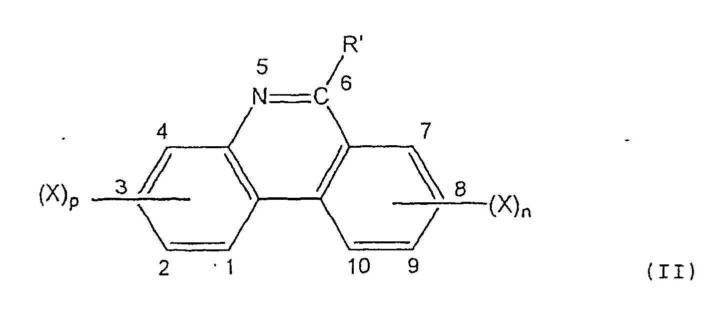 wobei
R' für eine Gruppe NH₂ steht;
X für F, Cl, CF₃ steht;
p und n gleich oder verschieden sind und gleich 0, 1 oder 2 sind;
in Verbindung mit wenigstens einem pharmazeutisch annehmbaren Träger.
